# EUROPÄISCHE PATENTSCHRIFT

(11) **EP 1 298 133 B1**
(45) Veröffentlichungstag und Bekanntmachung des Hinweises auf die Patenterteilung: **19.01.2005**
(21) Anmeldenummer: 02020594.4
(22) Anmeldetag: 18.09.2002
(51) Int. Cl.: C07D 497/04, C07D 333/38

(54) **Verfahren zur Alkylierung von 3,4-Dihydroxythiophen-2,5-dicarbonsäureestern**
Procedure for the alkylation of 3,4-dihydroxythiophene-2,5-dicarboxylic esters
Procédé pour la préparation des esters 3,4-dihydroxythiophène-2,5-dicarboxyliques

(30) Priorität: 01.10.2001 DE 10148437
(43) Veröffentlichungstag der Anmeldung: 02.04.2003
(73) Patentinhaber: Bayer Chemicals AG, 51368 Leverkusen (DE)
(72) Erfinder: Rauchschwalbe, Günter Dr., 51375 Leverkusen (DE); Klausener, Alexander Dr., 50259 Pulheim (DE); Bremen, Josef Dr., 51379 Leverkusen (DE); Schenkel, Ralf-Ingo Dr., 40591 Düsseldorf (DE); Winkler, Adolf Dr., 51373 Leverkusen (DE)

(56) Entgegenhaltungen:
- MERZ A ET AL: "IMPROVED PREPARATION OF 3,4-DIMETHOXYTHIOPHENE" JOURNAL FUR PRAKTISCHE CHEMIE, CHEMIKER ZEITUNG, WILEY VCH, WEINHEIM, DE, Bd. 338, 1996, Seiten 672-674, XP001000173 ISSN: 1436-9966
- COFFEY M ET AL: "A FACILE SYNTHESIS OF 3,4-DIALKOXYTHIOPHENES" SYNTHETIC COMMUNICATIONS, MARCEL DEKKER, INC., BASEL, CH, Bd. 26, Nr. 11, 1996, Seiten 2205-2212, XP001005513 ISSN: 0039-7911
- PEI Q ET AL: "ELECTROCHROMIC AND HIGHLY STABLE POLY (3,4-ETHYLENEDIOXYTHIOPHENE) SWITCHES BETWEEN OPAQUE BLUE-BLACK AND TRANSPARENT SKY BLUE" POLYMER, ELSEVIER SCIENCE PUBLISHERS B.V, GB, Bd. 35, Nr. 7, 1994, Seiten 1347-1351, XP001025972 ISSN: 0032-3861
- SANKARAAN B ET AL: "SYNTHESIS AND ELECTROCHEMISTRY OF POLYDIOXYETHIOPHENE AND ITS ALKYLSUBSTITUTED DERIVATIVES" POLYMERIC MATERIALS SCIENCE AND ENGINEERING, WASHINGTON, DC, US, Bd. 72, 1995, Seiten 319-320, XP001012695 ISSN: 0743-0515
- SCHOTTLAND P ET AL: "SYNTHESIS AND POLYMERIZATION OF NEW MONOMERS DERIVED FROM 3,4-ETHYLENEDIOXYTHIOPHENE" JOURNAL DE CHIMIE PHYSIQUE, SOCIETE DE CHIMIE PHYSIQUE, PARIS, FR, Bd. 95, Nr. 6, 1998, Seiten 1258-1261, XP001013494 ISSN: 0021-7689
- PAQUETTE, L. A. (ED.): "Encyclopedia of Reagents of Organic Synthesis, Volume 7" , J. WILEY & SONS , CHICHESTER XP002216647 Seiten 4724-4725: Tetra-n-butylammonium Bromide.
- RIDDIK J.A. ET AL: 'Organic Solvents, 4th ed.', 1986, WILEY, NEW YORK Pages 59-60, 67, 137-138.

## Beschreibung

Die Erfindung betrifft ein Verfahren zur Alkylierung von Dihydroxythiophen-dicarbonsäureestem bzw. ihrer Alkali- oder Erdalkalisalze in Gegenwart von organischen Oniumsalzen.

3,4-Dialkoxythiophene und 3,4-Alkylendioxythiophene sind Ausgangsverbindungen zur Herstellung von elektrisch leitfähigen, gegebenenfalls in dünner Schicht transparenten Polymeren, die in neuerer Zeit breite Verwendung finden. Die Polymere werden beispielsweise als Elektroden, Sensoren, zur Herstellung von Kondensatoren oder elektrolumineszierenden Displays und anderer elektrooptischer Bauteile, von photovoltaischen Vorrichtungen, als elektrochrome Schichten, als Hilfsmittel zur Herstellung von Metallüberzügen, als dünne Filme zur Ableitung statischer Aufladungen, in Gelelektrolyten oder in Ionenaustauschermembranen eingesetzt. Die Länge und das Substitutionsmuster der Alkoxy- bzw. Alkylengruppe gestattet es, die Eigenschaften dieser Polymere in weiten Grenzen zu variieren. Die Polymere werden im allgemeinen durch chemische oder elektrochemische Methoden aus den entsprechenden Monomeren hergestellt. Ein besonders wichtiges 3,4-Alkylendioxythiophen ist das 3,4-Ethylendioxythiophen.

3,4-Dialkoxythiophene und 3,4-Alkylendioxythiophene werden häufig in einer mehrstufigen Synthese aus den entsprechenden 3,4-Dialkoxythiophen-2,5-dicarbonsäuren und 3,4-Alkylendioxythiophen-2,5-dicarbonsäuren durch Decarboxylierung hergestellt, welche ihrerseits aus den entsprechenden Estern hergestellt werden. 3,4-Dialkoxythiophen-2,5-dicarbonsäureester und 3,4-Alkylendioxythiophen-2,5-dicarbonsäureester lassen sich durch Alkylierung aus 3,4-Dihydroxythiophen-2,5-dicarbonsäureestem herstellen. Während 3,4-Dihydroxythiophen-2,5-dicarbonsäureester bzw. ihre Alkali- und Erdalkalisalze seit langer Zeit in guter Ausbeute bequem erhalten werden können (s. beispielsweise O. Hinsberg, *Ber. Dt. Chem. Ges. 43,* **1910,** 901-906 und *45*, **1912**, 2413-2418) ist deren Alkylierung in vielen Fällen schwierig und nur mit mäßigen Ausbeuten durchzuführen.

Merz et al., *J. Prakt. Chem. 338,* **1996**, 672-674 beschreiben die Alkylierung von 3,4-Dihydroxythiophen-2,5-dicarbonsäureestern, welche mit Dimethylsulfat in Toluol in Gegenwart des Kationensolvators [18]Krone-6 durchgeführt wird, wobei die Ausbeute an freier Dicarbonsäure 73 % d. Theorie beträgt. Nachteilig an diesem Verfahren ist allerdings die lange Reaktionszeit von 48 Stunden. Weiterhin wird das Edukt in Form seines Dikaliumsalzes eingesetzt, welches gesondert aus dem Diol hergestellt werden muss. Die Alkylierung mit 1,2-Dichlorethan als Alkylierungsmittel, welche zu 3,4-Ethylendioxythiophen-2,5-dicarbonsäureester führen soll, welches vorzugsweise als Ausgangsstoff zur Herstellung von 3,4-Ethylendioxythiophen eingesetzt wird, gelang unter den genannten Bedingungen bei der Nachstellung im Labor nicht.

M. Coffey et al., *Synthetic Communications 26 (11)*, **1996**, 2205-2212 beschreiben die Alkylierung von 3,4-Dihydroxythiophen-2,5-dicarbonsäureestem in Form des freien Diols mit 1,2-Dibromethan in Anwesenheit von Kaliumcarbonat bei 150°C in Dimethylformamid. Nachteilig ist die niedrige Ausbeute von 52 % d. Theorie. Darüberhinaus ist die Herstellung des freien Diols aus dem primär erhaltenen Alkalisalz ein zusätzlicher Verfahrensschritt, der die Wirtschaftlichkeit der Synthese beeinträchtigt. Eine Variante dieser Verfahrensweise, bei der die Reaktionstemperatur 90°C beträgt, wurde von Sankaran und Reynolds, *Polym. Mater. SCI 72,* **1995**, 319-320 beschrieben.

Es bestand also Bedarf an einem einfach durchzuführenden Verfahren zur Alkylierung von 3,4-Dihydroxythiophen-2,5-dicarbonsäureestern bzw. deren Alkali- oder Erdalkalisalzen, welches bei vergleichsweise kurzen Reaktionszeiten gute Ausbeuten an den entsprechenden 3,4-Dialkoxythiophen-2,5-dicarbonsäureestern und 3,4-Alkylendioxythiophen-2,5-dicarbonsäureestern liefert.

Überraschenderweise wurde nun ein Verfahren zur Umsetzung von Verbindungen der Formel (I) 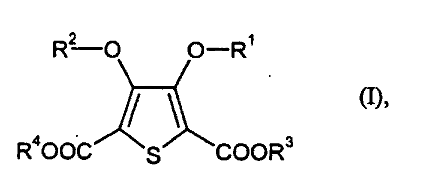 worin
- R¹ und R²: gleich sind und für Wasserstoff stehen oder gleich oder verschieden sind und für ein Alkalimetall oder ein Erdalkalimetall stehen und
- R³ und R⁴: gleich oder verschieden sind und für einen geradkettigen oder verzweigten Alkylrest mit 1 bis 10 Kohlenstoffatomen stehen,
mit Alkylierungsmitteln in einem polaren Verdünnungsmittel gefunden, welches dadurch gekennzeichnet ist, dass die Umsetzung in Gegenwart von quartären Oniumsalzen der Formel (II) 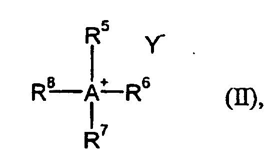 worin
- A: für Stickstoff oder Phosphor steht,
- Y⁻: für ein Anion steht und
- R⁵ bis R⁸: gleich oder verschieden sind und für einen Alkylrest mit 1 bis 20 Kohlenstoffatomen, einen Arylrest mit 6 bis 15 Kohlenstoffatomen oder einen Aralkylrest mit 7 bis 20 Kohlenstoffatomen stehen,
durchgeführt wird.

Das erfindungsgemäße Verfahren erlaubt die Alkylierung von Verbindungen der Formel (I) unter milden Bedingungen bei niedrigen Temperaturen und kurzen Reaktionszeiten, wobei ausgezeichnete Ausbeuten erhalten werden.

Vorzugsweise werden im erfindungsgemäßen Verfahren Verbindungen der Formel (I) eingesetzt, worin R¹ und R² gleich oder verschieden sind und für ein Alkalimetall oder ein Erdalkalimetall stehen, beispielsweise für Lithium, Natrium, Kalium, Rubidium, Magnesium, Calcium oder Strontium. Sie werden aus den entsprechenden freien 3,4-Dihydroxythiophen-2,5-dicarbonsäureestem durch Zusatz von Alkali- oder Erdalkalialkoholat oder -carbonat erhalten oder fallen direkt bei der Herstellung von 3,4-Dihydroxythiophen-2,5-dicarbonsäureester durch Umsetzung von Thiodiessigsäureester mit Oxalsäureester in Gegenwart von Alkalialkoholat ("Hinsberg-Kondensation") an Besonders bevorzugt werden Verbindungen der Formel (I) eingesetzt, worin R¹ und R² gleich oder verschieden sind und für ein Alkalimetall, beispielsweise Lithium, Natrium, Kalium, oder Rubidium stehen.

Werden Verbindungen der Formel (I) eingesetzt, worin R¹ und R² verschieden sind, so steht R¹ besonders bevorzugt für Natrium und R² besonders bevorzugt für Kalium.

Ganz besonders bevorzugt werden Verbindungen der Formel (I) eingesetzt, worin R¹ und R² gleich sind und für Lithium, Natrium, Kalium, oder Rubidium stehen, wobei hiervon Natrium und Kalium bevorzugt sind.

Vorzugsweise werden im erfindungsgemäßen Verfahren Verbindungen der Formel (I) eingesetzt, worin R³ und R⁴ gleich oder verschieden sind und für einen geradkettigen oder verzweigten Alkylrest mit 1 bis 8 Kohlenstoffatomen, besonders bevorzugt für einen geradkettigen oder verzweigten Alkylrest mit 1 bis 6 Kohlenstoffatomen wie beispielsweise Methyl, Ethyl, n-Propyl, iso-Propyl, n-Butyl, sec.-Butyl, tert.-Butyl, n-Pentyl, verzweigte Pentyle, n-Hexyl, verzweigte Hexyle, 2-Methylbutyl oder 2-Ethyl-butyl.

Werden im erfindungsgemäßen Verfahren Verbindungen der Formel (I) eingesetzt, worin R³ und R⁴ gleich sind, so stehen R³ und R⁴ vorzugsweise für Methyl, Ethyl, n-Propyl, iso-Propyl, n-Butyl, sec.-Butyl, tert.-Butyl, 2-Methyl-butyl oder 2-Ethylbutyl, besonders bevorzugt für Methyl, Ethyl, n-Propyl oder iso-Propyl, wobei die genannten Verbindungen besonders bevorzugt in Form ihrer Dinatrium- oder Dikaliumsalze (R¹ und R² sind gleich und stehen für Natrium oder Kalium) eingesetzt werden, ganz besonders bevorzugt in Form ihres Dinatriumsalzes (R¹ und R² sind gleich und stehen für Natrium). Ganz besonders bevorzugt wird der 3,4-Dihydroxythiophen-2,5-dicarbonsäuredimethylester in Form seines Dinatriumsalzes eingesetzt.

In einer bevorzugten Ausführungsform des erfindungsgemäßen Verfahrens werden Gemische von Verbindungen der Formel (I) eingesetzt, wie sie beispielsweise bei der Herstellung von 3,4-Dihydroxythiophen-2,5-dicarbonsäureester durch Umsetzung von Thiodiessigsäure-dibutylester mit Oxalsäure-diethylester in Gegenwart von Natriummethanolat in Methanol erhalten werden ("Hinsberg-Kondensation"). Dabei handelt es sich vorzugsweise um Gemische, worin R³ und R⁴ gleich oder unterschiedlich sind und für Methyl, Ethyl oder Butyl stehen. Besonders bevorzugt werden Gemische enthaltend 3,4-Dihydroxythiophen-2,5-dicarbonsäuredimethylester, 3,4-Dihydroxythiophen-2,5-dicarbonsäure-methyl-ethylester und 3,4-Dihydroxythiophen-2,5-dicarbonsäure-methyl-butylester eingesetzt. Ganz besonders bevorzugt werden Gemische in Form ihrer Salze eingesetzt (R¹ und R² sind gleich oder verschieden und stehen für ein Alkalimetall oder ein Erdalkalimetall), besonders bevorzugt sind dabei R¹ und R² gleich und stehen für Natrium oder Kalium, ganz besonders bevorzugt sind R¹ und R² gleich und stehen für Natrium. In einer ganz besonders bevorzugten Ausführungsform wird im erfindungsgemäßen Verfahren ein Gemisch, enthaltend das Dinatriumsalz des 3,4-Dihydroxythiophen-2,5-dicarbonsäuredimethylester, das Dinatriumsalz des 3,4-Dihydroxythiophen-2,5-dicarbonsäure-methyl-ethylester und das Dinatriumsalz des 3,4-Dihydroxythiophen-2,5-dicarbonsäure-methyl-butylester eingesetzt.

Bei den im erfindungsgemäßen Verfahren eingesetzten Alkylierungsmitteln handelt es sich um übliche, dem Fachmann bekannte Alkylierungsmittel.

Dabei können Alkylierungsmittel eingesetzt werden, welche nur eine Abgangsgruppe enthalten, wobei diese bei Umsetzung mit den im erfindungsgemäßen Verfahren eingesetzten Verbindungen der Formel (I) zu Verbindungen der Formel (III) führen 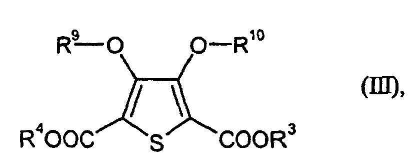 worin
- R⁹ und R¹⁰: für geradkettige oder verzweigte Alkylreste stehen, welche aus dem jeweiligen verwendeten Alkylierungsmittel stammen und
- R³ und R⁴: die oben genannte Bedeutung haben.

Vorzugsweise werden dabei Alkylierungsmittel aus der Reihe Alkylhalogenide, Alkylsulfate, Alkylmethansulfonate, Alkylbenzolsulfonate und Alkyltoluolsulfonate eingesetzt, vorzugsweise geradkettige oder verzweigte C₁-C₈-Alkylhalogenide, -sulfate, -methansulfonate, -benzolsulfonate und -toluolsulfonate. Besonders bevorzugt werden Alkylhalogenide eingesetzt, vorzugsweise geradkettige oder verzweigte C₁-C₈-Alkylhalogenide, ganz besonders bevorzugt geradkettige oder verzweigte C₁-C₈-Alkylchloride oder -bromide, insbesondere Dimethylsulfat, Methylchlorid, Methylbromid oder Methyliodid.

Bevorzugt werden Alkylierungsmittel eingesetzt, welche zwei Abgangsgruppen enthalten, wobei diese bei Umsetzung mit den im erfindungsgemäßen Verfahren eingesetzten Verbindungen der Formel (I) zu Verbindungen der Formel (IV) führen 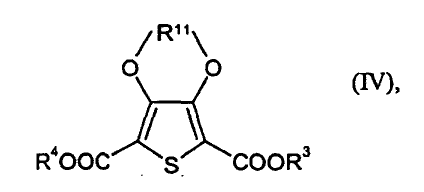 worin
- R¹¹: für einen geradkettigen oder verzweigten Alkylenrest steht, welcher aus dem jeweiligen verwendeten Alkylierungsmittel stammt und
- R³ und R⁴: die oben genannte Bedeutung haben.

Vorzugsweise werden dabei Alkylierungsmittel aus der Reihe Alkyldihalogeniden, Alkyldisulfaten, Alkyldimethansulfonaten, Alkyldibenzolsulfonaten und Alkylditoluolsulfonaten eingesetzt, vorzugsweise geradkettige oder verzweigte C₁-C₈-Alkyldihalogenide, -disulfate, -dimethansulfonate, -dibenzolsulfonate und -ditoluolsulfonate. Besonders bevorzugt werden Alkyldihalogenide eingesetzt, vorzugsweise geradkettige oder verzweigte C₁-C₈-Alkyldihalogenide, ganz besonders bevorzugt geradkettige oder verzweigte C₁-C₈-Alkyldichloride oder -dibromide, insbesondere 1,2-Bis-mesyloxyethan, 1,2-Dichlorethan, 1,2-Dibromethan und 1-Brom-2-chlorethan, hiervon ganz besonders bevorzugt 1,2-Dichlorethan.

Das im erfindungsgemäßen Verfahren eingesetzte Alkylierungsmittel wird wenigstens in stöchiometrischer Menge eingesetzt, im Allgemeinen ist es vorteilhaft, einen Überschuss einzusetzen. Vorzugsweise wird ein Überschuss von 30 bis 200 mol-% verwendet, besonders bevorzugt ein Überschuss von 50 bis 150 mol-%, insbesondere ein Überschuss von 70 bis 100 mol-%, bezogen auf die Verbindungen der Formel (I).

Bei den im erfindungsgemäßen Verfahren eingesetzten polaren Verdünnungsmitteln handelt es sich um übliche, dem Fachmann bekannte polaren Verdünnungsmittel. Diese können allein oder in Form von Gemischen eingesetzt werden. Es ist weiterhin möglich, polaren Verdünnungsmittel in Form von Mischungen mit unpolaren Verdünnungsmitteln einzusetzen.

Vorzugsweise werden als polare Verdünnungsmittel aprotische polare Verdünnungsmittel aus der Reihe Ether, Ketone, Ester, Amide, Harnstoffe, Sulfoxide und Sulfone eingesetzt, besonders bevorzugt Diethylenglykol-dimethylether, Dioxan, Aceton, Cyclohexanon, Essigsäure-butylester, N,N-Dimethylformamid, N-Methylpyrrolidon, Hexamethyl-phosphorsäureamid, Tetramethylharnstoff, Dimethylsulfoxid oder Tetramethylen-sulfon (Sulfolan).

Vorzugsweise werden im erfindungsgemäßen Verfahren quartäre Oniumsalze der Formel (II) eingesetzt, worin Y- für ein Anion aus der Reihe Chlorid, Bromid, Iodid, Hydroxid, Hydrogensulfat, Sulfat, Methansulfonat, Toluolsulfonat und Trifluoracetat steht, besonders bevorzugt für Chlorid oder Bromid.

Vorzugsweise werden im erfindungsgemäßen Verfahren quartäre Oniumsalze der Formel (II) eingesetzt, worin R⁵ bis R⁸ gleich oder verschieden sind und vorzugsweise für einen Alkylrest mit 1 bis 16 Kohlenstoffatomen, einen Arylrest mit 6 bis 10 Kohlenstoffatomen wie beispielsweise Phenyl oder Naphthyl oder einen Aralkylrest mit 7 bis 11 Kohlenstoffatomen stehen, wie beispielsweise Benzyl, Phenylmethyl oder Phenylethyl, besonders bevorzugt für Methyl, Ethyl, n-Propyl, iso-Propyl, n-Butyl, sec.-Butyl, tert.-Butyl, Decyl, Dodecyl, Tetradecyl, Phenyl oder Benzyl.

Besonders bevorzugt werden im erfindungsgemäßen Verfahren quartäre Oniumsalze der Formel (II) eingesetzt, worin R⁵ bis R⁸ gleich sind und für einen Alkylrest mit 1 bis 6 Kohlenstoffatomen wie beispielsweise Methyl, Ethyl, n-Propyl, iso-Propyl, n-Butyl, sec.-Butyl, tert.-Butyl, n-Pentyl, verzweigte Pentyle, n-Hexyl oder verzweigte Hexyle stehen, besonders bevorzugt für Methyl, Ethyl, n-Propyl, iso-Propyl, n-Butyl, sec.-Butyl oder tert.-Butyl.

Ganz besonders bevorzugt werden im erfindungsgemäßen Verfahren quartäre Oniumsalze der Formel (II) aus der Reihe Tetramethylammoniumchlorid, Tetramethylammoniumbromid, Tetrabutylammoniumchlorid, Tetrabutylammoniumbromid, Benzyl-dimethyl-tetradecylammoniumchlorid, Butyl-triphenylphosphoniumbromid, Tetraphenylphosphoniumbromid, Tetrabutylphosphoniumbromid und Tetrabutylphosphoniumchlorid eingesetzt.

Die im erfindungsgemäßen Verfahren eingesetzten quartären Oniumsalze der Formel (II) können einzeln oder in Form von Mischungen verschiedener Oniumsalze eingesetzt werden. Weiterhin können sie als solche eingesetzt werden oder in Form von Vorläuferverbindungen eingesetzt werden, welche sich unter den Bedingungen des erfindungsgemäßen Verfahrens *in situ* zu Oniumsalzen der Formel (II) umsetzen, wie beispielsweise tertiäre Amine oder Phosphine, welche unter den Reaktionsbedingungen zu quartären Ammoniumsalzen bzw. Phosphoniumsalzen alkyliert werden.

Die im erfindungsgemäßen Verfahren eingesetzten Oniumsalze werden vorzugsweise in unterstöchiometrischer Menge verwendet, bevorzugt in einer Menge von 1 bis 20 mol-%, bezogen auf Dihydroxythiophenderivat (Edukt), besonders bevorzugt von 2 bis 10 mol-%, ganz besonders bevorzugt in einer Menge von 3 bis 7 mol-%, bezogen auf Dihydroxythiophenderivat (Edukt).

In einer bevorzugten Ausführungsform wird das erfindungsgemäße Verfahren in Gegenwart einer zusätzlichen Base durchgeführt, besonders bevorzugt in Gegenwart von Alkali - oder Erdalkalimetallcarbonaten, -hydroxiden, -oxiden oder Alkoholaten, ganz besonders bevorzugt in Gegenwart von Alkalimetallcarbonaten, insbesondere von Natriumcarbonat oder Kaliumcarbonat.

Wird in Gegenwart einer zusätzlichen Base gearbeitet, so wird diese vorzugsweise in unterstöchiometrischer bis äquivalenter Menge eingesetzt, bevorzugt von 5 bis 100 mol-%, besonders bevorzugt von 10 bis 80 mol-%, ganz besonders bevorzugt von 25 bis 50 mol-%, bezogen auf die Verbindungen der Formel (I).

Das erfindungsgemäße Verfahren wird vorzugsweise bei einer Temperatur von 50 bis 150°C durchgeführt. Wird 1,2-Dichlorethan oder 1,2-Dibromethan als Alkylierungsmittel eingesetzt, so wird vorzugsweise bei einer Temperatur von 100 bis 140°C gearbeitet.

Das erfindungsgemäße Verfahren wird vorzugsweise bei Normaldruck oder erhöhtem Druck, vorzugsweise bei 1 bis 30 bar, durchgeführt. Das Arbeiten unter erhöhtem Druck erlaubt den Einsatz von Verdünnungsmitteln, deren Siedepunkt bei Normaldruck wesentlich unter der gewünschten Verfahrenstemperatur liegt.

Das erfindungsgemäße Verfahren wird vorzugsweise so durchgeführt, dass das Verdünnungsmittel vorlegt wird und zu diesem vorzugsweise zuerst Verbindungen der Formel (I), quartäre Oniumsalze der Formel (II) und vorzugsweise eine Base zugibt. Das Gemisch wird vorzugsweise auf die gewünschte Reaktionstemperatur gebracht und anschließend wird das Alkylierungsmittel zugegeben, vorzugsweise durch Zutropfen. Nach Beendigung der Reaktion erhält man als Produkte Verbindungen der Formel (III) oder (IV). Diese können durch Austragen auf Wasser, Abfiltrieren und Umkristallisieren isoliert und aufgereinigt werden. In einer bevorzugten Ausführungsform wird auf die Isolierung und Aufreinigung dieser Verbindungen verzichtet. Die Verbindungen der Formel (III) oder (IV) werden, vorzugsweise nach Entfernung mindestens eines Teils des Verdünnungsmittels, beispielsweise durch Destillation, nach den in der Literatur bekannten Methoden zu den entsprechenden Carbonsäuren verseift. Diese können beispielsweise nach Ansäuern des Reaktionsgemisches durch Filtration oder Zentrifugation isoliert werden.

### Beispiele

### Beispiel 1

In einem 1-Liter Rührkolben mit Rückflusskühler wurden 135 g N,N-Dimethylformamid vorgelegt. Hierzu wurden 55,2 g (0,2 mol) 3,4-Dihydroxythiophen-2,5-dicarbonsäuredimethylester in Form seines Dinatriumsalzes, 10,8 g Kaliumcarbonat und 3,4 g Tetrabutylammoniumbromid gegeben. Die Mischung wurde unter Stickstoff auf eine Temperatur von 130 bis 135°C erhitzt. Sobald die Reaktionstemperatur erreicht wurde, wurden innerhalb von 15 min 39 g (0.4 mol) Dichlorethan zugetropft. Nach Beendigung des Zutropfens wurde für weitere 5 h gerührt. Anschließend wurde das Verdünnungsmittel unter Vakuum abdestilliert (90 % Wiedergewinnung des Verdünnungsmittels).

Nun wurden 500 ml Wasser, 40 g 2-Propanol und 43 g Natriumhydroxid (45 %ige wässrige Lösung) zugesetzt, das Reaktionsgemisch auf 80°C erwärmt und für 2 h gerührt. Anschließend wurde das Reaktionsgemisch durch Zutropfen von 70 bis 80 ml HCl angesäuert, wobei das Produkt ausfiel. Das ausgefallene Produkt wurde abgesaugt und dreimal mit je 90 ml Wasser gewaschen. Das feuchte Produkt wurde im Vakuumtrockenschrank (200 mbar / 50°C) getrocknet.

Man erhielt 38,5 g 3,4-Ethylendioxythiophen-2,5-dicarbonsäure in einer Reinheit von 91,9 % (HPLC-Analyse), was einer Ausbeute von 76,9 % d. Theorie entspricht.

### Vergleichsbeispiel 1

Das Vergleichsbeispiel wurde analog zu Beispiel 1, jedoch ohne Zusatz von Tetrabutylammoniumbromid durchgeführt. Dadurch ergab sich eine Reaktionszeit von 12 h bis zum vollständigen Umsatz (durch HPLC-Analyse von Zwischenproben bestimmt). Man erhielt 34,4 g 3,4-Ethylendioxythiophen-2,5-dicarbonsäure in einer Reinheit von 86,9 % (HPLC-Analyse), was einer Ausbeute von 67 % d. Theorie entspricht.

### Beispiel 2

Beispiel 2 wurde analog zu Beispiel 1 durchgeführt, wobei statt des Dinatriumsalzes des 3,4-Dihydroxythiophen-2,5-dicarbonsäuredimethylesters 0,177 mol eines Gemisches aus 3,4-Dihydroxythiophen-2,5-dicarbonsäuredimethylester, 3,4-Dihydroxythiophen-2,5-dicarbonsäure-methyl-ethylester und 3,4-Dihydroxythio-phen-2,5-dicarbonsäure-methy-butylester (Gewichtsverhältnis 10 : 1 : 14) in Form ihrer Dinatriumsalze eingesetzt wurde.

Es wurde 3,4-Ethylendioxythiophen-2,5-dicarbonsäure in einer Ausbeute von 76,8 % d. Theorie isoliert.

### Beispiel 3 bis 7

Die Beispiele 3 bis 7 wurden analog zu Beispiel 2 durchgeführt, wobei die Art des eingesetzten Oniumsalzes variiert wurde. Die Ergebnisse sind in Tabelle 1 dargestellt.

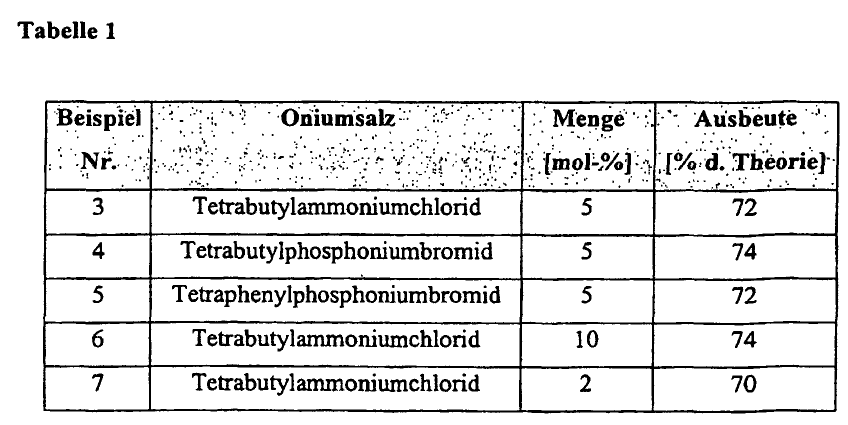

### Beispiel 8 (Durchführung ohne Basenzusatz)

In einem Rührkolben mit Rückflusskühler wurden 590 ml N,N-Dimethylformamid vorgelegt. Hierzu wurden 137,3 g (0,42 mol) des in Beispiel 2 beschriebenen Estergemisches und 8,1 g Tetrabutylammoniumbromid gegeben. Die Mischung wurde unter Stickstoff auf eine Temperatur von 80°C erwärmt, dann wurden 96 g 1,2-Dichlorethan zugetropft und die Reaktionsmischung wurde auf 135°C erhitzt. Nach Beendigung des Zutropfens wurde für weitere 5 h gerührt. Anschließend wurde das Verdünnungsmittel unter Vakuum (26 mbar) bei 80°C abdestilliert.

Nun wurden 750 ml Wasser, 81,5 ml Natriumhydroxid (50 %ige wässrige Lösung) zugesetzt, das Reaktionsgemisch auf 98°C erwärmt und für 5 h gerührt. Anschließend wurde das Reaktionsgemisch durch Zutropfen von Schwefelsäure auf einen pH von ca. 1 angesäuert, wobei das Produkt ausfiel. Das ausgefallene Produkt wurde abgesaugt und mit insgesamt 1000 ml Wasser gewaschen. Das feuchte Produkt wurde im Vakuumtrockenschrank (200 mbar / 50°C) getrocknet.

Man erhielt 3,4-Ethylendioxythiophen-2,5-dicarbonsäure in einer Ausbeute von 66 % d. Theorie.

### Beispiel 9

Beispiel 9 wurde analog zu Beispiel 2 in einem Autoklaven unter Druck durchgeführt.

Man erhielt 3,4-Ethylendioxythiophen-2,5-dicarbonsäure in einer Ausbeute von 74 % d. Theorie.

### Beispiel 10

In einem Rührkolben mit Rückflusskühler wurden 455 ml N,N-Dimethylformamid vorgelegt. Hierzu wurden 186 g (0,569 mol) des in Beispiel 2 beschriebenen Estergemisches, 22 g Tetrabutylammoniumbromid und 35 g Kaliumcarbonat gegeben. Die Mischung wurde unter Stickstoff auf eine Temperatur von 125°C erwärmt, dann wurden 121 g 1,2-Dichlorethan zugetropft. Nach Beendigung des Zutropfens wurde für weitere 2 h gerührt bei 125°C und für 1 h bei 135°C gerührt. Anschließend wurde das Verdünnungsmittel unter Vakuum (20 mbar) bei 60°C abdestilliert (Wiedergewinnung des Verdünnungsmittels > 90 %).

Nun wurden 500 ml Wasser,120 g Propanol und 120 g Natriumhydroxid (45 %ige wässrige Lösung) zugesetzt, das Reaktionsgemisch auf 80 bis 85°C erwärmt und für 0,5 h gerührt. Anschließend wurden weitere 500 ml Wasser zugesetzt und für weitere 0,5 h gerührt. Zum Ausfällen des Reaktionsproduktes wurden 234 ml konz. Salzsäure und 234 ml Wasser vorgelegt und auf 50°C erwärmt. Das Reaktionsgemisch wurde unter Rühren innerhalb von 4 h zugetropft, wobei am Ende des Zutropfen ein pH von ca. 1 vorlag und das Produkt ausfiel. Die Mischung wurde auf 30°C abgekühlt. Das ausgefallene Produkt wurde schnell abgesaugt und rasch mit Wasser gewaschen. Das feuchte Produkt wurde im Vakuumtrockenschrank (100 bis 200 mbar / 50 bis 60°C) getrocknet.

Man erhielt 107,2 g 3,4-Ethylendioxythiophen-2,5-dicarbonsäure in einer Reinheit von 92,5 % (HPLC-Analyse), was einer Ausbeute von 75,7 % d. Theorie entspricht.

### Beispiel 11

Die gemäß Beispiel 1 hergestellte 3,4-Ethylendioxythiophen-2,5-dicarbonsäure wurde durch Lösen in wässriger Natronlauge, Behandeln mit Aktivkohle und Ausfällen mit Salzsäure nachgereinigt.

Durch Elementaranalyse wurden folgende Ergebnisse enthalten:

| | |
|---|---|
| C (gefunden) | 38,8 % |
| H (gefunden) | 3,15 % |
| S (gefunden) | 12,9 % |

| | |
|---|---|
| C (berechnet für Monohydrat) | 38,7 % |
| H (berechnet für Monohydrat) | 3,25 % |
| S (berechnet für Monohydrat) | 12,9 % |

| | |
|---|---|
| C (berechnet für wasserfreie Verbindung) | 41,7 % |
| H (berechnet für wasserfreie Verbindung) | 2,63 % |
| S (berechnet für wasserfreie Verbindung) | 13,9 % |

Die Elementaranalyse liefert korrekte Werte für 3,4-Ethylendioxythiophen-2,5-dicarbonsäure-Monohydrat. Durch Trocknen bei 120°C kann das Kristallwasser entzogen werden, das wasserfreie Produkt zieht an feuchter Luft wieder allmählich Wasser.

### Beispiel 12

In einem Rührkolben mit Rückflusskühler wurden 600 ml Aceton vorgelegt. Hierzu wurden 248,5 g (0,9 mol) 3,4-Dihydroxythiophen-2,5-dicarbonsäuredimethylester in Form seines Dinatriumsalzes und 10 g Tetrabutylammoniumbromid gegeben. Die Mischung wurde zum Sieden erhitzt und innerhalb von 4 h wurden 600 g Dimethylsulfat zugetropft. Nach Beendigung des Zutropfens wurde für weitere 2 h gerührt. Anschließend wurde die Reaktionsmischung zu 3 Liter Wasser zugegeben, der dabei ausfallende Feststoff wurde abgesaugt und mit Wasser gewaschen. Das feuchte Produkt wurde im Vakuumtrockenschrank (100 bis 200 mbar / 50 bis 60°C) getrocknet.

Man erhielt 235 g 3,4-Dimethoxythiophen-2,5-dicarbonsäuredimethylester in einer Ausbeute von 85,5 % d. Theorie. Durch alkalische Verseifung, Decarboxylierung und Destillation konnte 3,4-Dimethoxythiophen erhalten werden.

### Vergleichsbeispiel 12

Das Vergleichsbeispiel 12 wurde analog zu Beispiel 12, jedoch ohne Zusatz von Tetrabutylammoniumbromid durchgeführt.

Man erhielt 235 g 3,4-Dimethoxythiophen-2,5-dicarbonsäuredimethylester in einer Ausbeute von 65 % d. Theorie.

## Patentansprüche

1. Verfahren zur Umsetzung von Verbindungen der Formel (I) 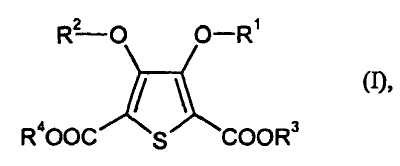 worin
R¹ und R² gleich sind und für Wasserstoff stehen oder gleich oder verschieden sind und für ein Alkalimetall oder ein Erdalkalimetall stehen und
R³ und R⁴ gleich oder verschieden sind und für einen geradkettigen oder verzweigten Alkylrest mit 1 bis 10 Kohlenstoffatomen stehen,
mit Alkylierungsmitteln in einem polaren Verdünnungsmittel, welches **dadurch gekennzeichnet ist, dass** die Umsetzung in Gegenwart von quartären Oniumsalzen der Formel (II) 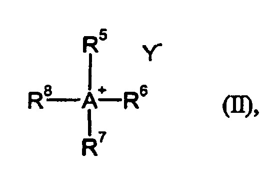 worin
A für Stickstoff oder Phosphor steht,
Y⁻ für ein Anion steht und
R⁵ bis R⁸ gleich oder verschieden sind und für einen Alkylrest mit 1 bis 20 Kohlenstoffatomen, einen Arylrest mit 6 bis 15 Kohlenstoffatomen oder einen Aralkylrest mit 7 bis 20 Kohlenstoffatomen stehen,
durchgeführt wird.

2. Verfahren gemäß Anspruch 1, **dadurch gekennzeichnet, dass** als Verbindungen der Formel (I) solche eingesetzt werden, worin R¹ und R² gleich oder verschieden sind und für ein Alkalimetall oder ein Erdalkalimetall stehen und R³ und R⁴ gleich oder verschieden sind und für einen geradkettigen oder verzweigten Alkylrest mit 1 bis 8 Kohlenstoffatomen stehen.

3. Verfahren gemäß einem oder mehreren der vorangegangenen Ansprüche 1 und 2, **dadurch gekennzeichnet, dass** als Verbindungen der Formel (I) solche eingesetzt werden, worin R¹ und R² gleich sind und für ein Alkalimetall stehen und R³ und R⁴ gleich oder verschieden sind und für einen geradkettigen oder verzweigten Alkylrest mit 1 bis 8 Kohlenstoffatomen stehen.

4. Verfahren gemäß einem oder mehreren der vorangegangenen Ansprüche 1 bis 3, **dadurch gekennzeichnet, dass** als Verbindungen der Formel (I) 3,4-Dihydroxythiophen-2,5-dicarbonsäuredimethylester in Form seines Dinatrium- oder Dikaliumsalzes eingesetzt wird.

5. Verfahren gemäß einem oder mehreren der vorangegangenen Ansprüche 1 bis 4, **dadurch gekennzeichnet, dass** Gemische aus Verbindungen der Formel (I) eingesetzt werden.

6. Verfahren gemäß einem oder mehreren der vorangegangenen Ansprüche 1 bis 5, **dadurch gekennzeichnet, dass** als Verbindungen der Formel (I) ein Gemisch enthaltend das Dinatriumsalz des 3,4-Dihydroxythiophen-2,5-dicarbon-säuredünethylester, das Dinatriumsalz des 3,4-Dihydroxythiophen-2,5-dicarbon-säure-methyl-ethylester und das Dinatriumsalz des 3,4-Dihydroxythiophen-2,5-dicarbonsäure-methyl-butylester, eingesetzt wird.

7. Verfahren gemäß einem oder mehreren der vorangegangenen Ansprüche 1 bis 6, **dadurch gekennzeichnet, dass** als Alkylierungsmittel ein Alkylierungsmittel aus der Reihe Alkylhalogenide, Alkylsulfate, Alkylmethansulfonate, Alkylbenzolsulfonate Alkyltoluolsulfonate, Alkyldihalogenide, Alkyldisulfate, Alkyldimethansulfonate, Alkyldibenzolsulfonate und Alkylditoluolsulfonate eingesetzt wird.

8. Verfahren gemäß einem oder mehreren der vorangegangenen Ansprüche 1 bis 7, **dadurch gekennzeichnet, dass** als Alkylierungsmittel ein Alkylierungsmittel aus der Reihe C₁-C₈-Alkylhalogenid und C₁-C₈-Alkyldihalogenid eingesetzt wird.

9. Verfahren gemäß einem oder mehreren der vorangegangenen Ansprüche 1 bis 8, **dadurch gekennzeichnet, dass** als polares Verdünnungsmittel ein polares aprotisches Verdünnungsmittel, vorzugsweise ein polares aprotisches Verdünnungsmittel aus der Reihe Ether, Ketone, Ester, Amide, Harnstoffe, Sulfoxide und Sulfone eingesetzt wird.

10. Verfahren gemäß einem oder mehreren der vorangegangenen Ansprüche 1 bis 9, **dadurch gekennzeichnet, dass** als quartäre Oniumsalze solche der Formel (II) eingesetzt werden, worin
A für Stickstoff oder Phosphor steht,
Y⁻ für ein Anion aus der Reihe Chlorid, Bromid, Iodid, Hydroxid, Hydrogensulfat, Sulfat, Methansulfonat, Toluolsulfonat und Trifluoracetat steht und
R⁵ bis R⁸ gleich oder verschieden sind und für einen Alkylrest mit 1 bis 16 Kohlenstoffatomen, einen Arylrest mit 6 bis 10 Kohlenstoffatomen oder einen Aralkylrest mit 7 bis 11 Kohlenstoffatomen stehen.

11. Verfahren gemäß einem oder mehreren der vorangegangenen Ansprüche 1 bis 10, **dadurch gekennzeichnet, dass** als quartäre Oniumsalze ein quartäres Oniumsalz aus der Reihe Tetramethylammoniumchlorid, Tetramethylammoniumbromid, Tetrabutylammoniumchlorid, Tetrabutylammoniumbromid, Benzyl-dimethyltetradecylammonium-chlorid, Butyl-triphenylphosphoniumbromid, Tetraphenylphosphoniumbromid, Tetrabutylphosphoniumbromid und Tetrabutylphosphoniumchlorid eingesetzt wird.

12. Verfahren gemäß einem oder mehreren der vorangegangenen Ansprüche 1 bis 11, **dadurch gekennzeichnet, dass** es in Gegenwart einer zusätzlichen Base durchgeführt wird.

13. Verfahren gemäß einem oder mehreren der vorangegangenen Ansprüche 1 bis 12, **dadurch gekennzeichnet, dass** es bei einer Temperatur von 50 bis 150°C durchgeführt wird.

## Claims

1. Process for the reaction of compounds of the formula (I) 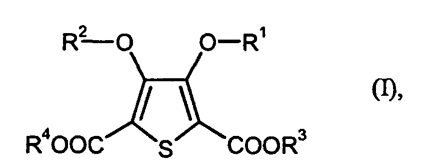 where
R¹ and R² are identical and are each hydrogen or are identical or different and are each an alkali metal or an alkaline earth metal and
R³ and R⁴ are identical or different and are each a straight-chain or branched alkyl radical having from 1 to 10 carbon atoms,
with alkylating agents in a polar diluent, which is **characterized in that** the reaction is carried out in the presence of quaternary onium salts of the formula (II) 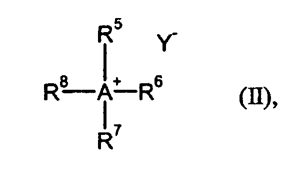 where
A is nitrogen or phosphorus,
Y⁻ is an anion and
R⁵ to R⁸ are identical or different and are each an alkyl radical having from 1 to 20 carbon atoms, an aryl radical having from 6 to 15 carbon atoms or an aralkyl radical having from 7 to 20 carbon atoms.

2. Process according to Claim 1, **characterized in that** the compounds of the formula (I) used are ones in which R¹ and R² are identical or different and are each an alkali metal or an alkaline earth metal and R³ and R⁴ are identical or different and are each a straight-chain or branched alkyl radical having from 1 to 8 carbon atoms.

3. Process according to Claim 1 or 2, **characterized in that** the compounds of the formula (I) used are ones in which R¹ and R² are identical and are each an alkali metal and R³ and R⁴ are identical or different and are each a straight-chain or branched alkyl radical having from 1 to 8 carbon atoms.

4. Process according to one or more of Claims 1 to 3, **characterized in that** dimethyl 3,4-dihydroxythiophene-2,5-dicarboxylate in the form of its disodium or dipotassium salt is used as compounds of the formula (I).

5. Process according to one or more of Claims 1 to 4, **characterized in that** the mixtures of compounds of the formula (I) are used.

6. Process according to one or more of Claims 1 to 5, **characterized in that** a mixture comprising the disodium salt of dimethyl 3,4-dihydroxythiophene-2,5-dicarboxylate, the disodium salt of methyl ethyl 3,4-dihydroxythiophene-2,5-dicarboxylate and the disodium salt of methyl butyl 3,4-dihydroxythiophene-2,5-dicarboxylate is used as compounds of the formula (I).

7. Process according to one or more of Claims 1 to 6, **characterized in that** the alkylating agent used is an alkylating agent selected from the group consisting of alkyl halides, alkyl sulphates, alkyl methane sulphonates, alkyl benzene sulphonates, alkyl toluene sulphonates, alkyl dihalides, alkyl disulphates, alkyl dimethane sulphonates, alkyl dibenzene sulphonates and alkyl ditoluene sulphonates.

8. Process according to one or more of Claims 1 to 7, **characterized in that** the alkylating agent used is an alkylating agent selected from the group consisting of C₁-C₈-alkyl halides and C₁-C₈-alkyl dihalides.

9. Process according to one or more of Claims 1 to 8, **characterized in that** the polar diluent used is a polar aprotic diluent, preferably a polar aprotic diluent selected from the group consisting of ethers, ketones, esters, amides, ureas, sulphoxides and sulphones.

10. Process according to one or more of Claims 1 to 9, **characterized in that** the quaternary onium salts used are ones of the formula (II) in which
A is nitrogen or phosphorus,
Y⁻ is an anion selected from the group consisting of chloride, bromide, iodide, hydroxide, hydrogen sulphate, sulphate, methane sulphonate, toluene sulphonate and trifluoroacetate and
R⁵ to R⁸ are identical or different and are each an alkyl radical having from 1 to 16 carbon atoms, an aryl radical having from 6 to 10 carbon atoms or an aralkyl radical having from 7 to 11 carbon atoms.

11. Process according to one or more of Claims 1 to 10, **characterized in that** the quaternary onium salt used is a quaternary onium salt selected from the group consisting of tetramethylammonium chloride, tetramethylammonium bromide, tetrabutylammonium chloride, tetrabutyl ammonium bromide, benzyldimethyltetradecylammonium chloride, butyltriphenylphosphonium bromide, tetraphenylphosphonium bromide, tetrabutylphosphonium bromide and tetrabutylphosphonium chloride.

12. Process according to one or more of Claims 1 to 11, **characterized in that** it is carried out in the presence of an additional base.

13. Process according to one or more of Claims 1 to 12, **characterized in that** it is carried out at a temperature of from 50 to 150°C.

## Revendications

1. Procédé destiné à faire réagir des composés de formule (I) 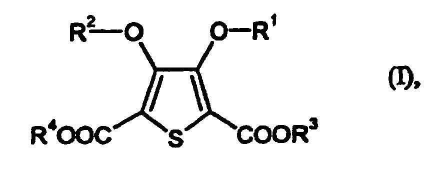 dans laquelle
R¹ et R² sont identiques et représentent un atome d'hydrogène, ou sont identiques ou différents et représentent un métal alcalin ou un métal alcalino-terreux, et
R³ et R⁴ sont identiques ou différents et représentent un reste alkyle ramifié ou à chaîne droite ayant 1 à 10 atomes de carbone,
avec des agents d'alkylation dans un diluant polaire, lequel est **caractérisé en ce que** la réaction se produit en présence de sels d'onium quaternaires de formule (II) 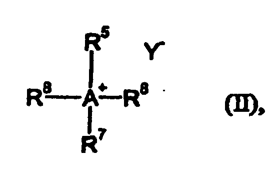 dans laquelle
A représente un azote ou un phosphore,
Y⁻ représente un anion,
R⁵ à R⁸ sont identiques ou différents et représentent un reste alkyle ayant 1 à 20 atomes de carbone, un reste aryle ayant 6 à 15 atomes de carbone ou un reste aralkyle ayant 7 à 20 atomes de carbone.

2. Procédé selon la revendication 1, **caractérisé en ce que** comme composés de formule (I), on utilise ceux dans lesquels R₁ et R₂ sont identiques ou différents et représentent un métal alcalin ou un métal alcalino-terreux, et R³ et R⁴ sont identiques ou différents et représentent un reste alkyle ramifié ou à chaîne droite ayant 1 à 8 atomes de carbone.

3. Procédé selon l'une quelconque ou plusieurs des revendications précédentes 1 et 2, **caractérisé en ce que** comme composés de formule (I), on utilise ceux dans lesquels R¹ et R² sont identiques et représentent un métal alcalin, et R³ et R⁴ sont identiques ou différents et représentent un reste alkyle ramifié ou à chaîne droite ayant 1 à 8 atomes de carbone.

4. Procédé selon l'une quelconque ou plusieurs des revendications précédentes 1 à 3, **caractérisé en ce que** comme composés de formule (I), on utilise l'ester diméthylique d'acide 3,4-dihydroxythiophène-2,5-dicarboxylique sous la forme d'un sel de disodium ou de dipotassium.

5. Procédé selon l'une quelconque ou plusieurs des revendications précédentes 1 à 4, **caractérisé en ce que** l'on utilise des mélanges de composés de formule (I).

6. Procédé selon l'une quelconque ou plusieurs des revendications précédentes 1 à 5, **caractérisé en ce que** comme composés de formule (I), on utilise un mélange contenant le sel de disodium d'ester diméthylique d'acide 3,4-dihydroxythiophène-2,5-dicarboxylique, le sel de disodium d'ester de méthyléthyle d'acide 3,4-dihydroxythiophène-2,5-dicarboxylique et le sel de disodium d'ester de méthylbutyle d'acide 3,4-dihydroxythiophène-2,5-dicarboxylique.

7. Procédé selon l'une quelconque ou plusieurs des revendications précédentes 1 à 6, **caractérisé en ce que** comme agent d'alkylation, on utilise un agent d'alkylation choisi parmi l'halogénure d'alkyle, le sulfate d'alkyle, le méthanesulfonate d'alkyle, le sulfonate d'alkylbenzène, le toluène sulfonate d'alkyle, le dihalogénure d'alkyle, le disulfate d'alkyle, le diméthanesulfonate d'alkyle, le disulfonate d'alkylbenzène et le toluène disulfonate d'alkyle.

8. Procédé selon l'une quelconque ou plusieurs des revendications précédentes 1 à 7, **caractérisé en ce que** comme agent d'alkylation, on utilise un agent d'alkylation choisi parmi l'halogénure d'alkyle en C₁-C₈ et le dihalogénure d'alkyle en C₁-C₈.

9. Procédé selon l'une quelconque ou plusieurs des revendications précédentes 1 à 8, **caractérisé en ce que** comme diluant polaire, on utilise un diluant aprotique polaire, de préférence un diluant aprotique polaire choisi parmi les éthers, les cétones, les esters, les amides, les urées, les sulfoxydes et les sulfones.

10. Procédé selon l'une quelconque ou plusieurs des revendications précédentes 1 à 9, **caractérisé en ce que** comme sels d'onium quaternaires, on utilise ceux de formule (II) dans laquelle
A représente un azote et/ou un phosphore,
Y⁻ représente un anion choisi parmi le chlorure, le bromure, l'iodure, l'hydroxyde, l'hydrogénosulfate, le sulfate, le méthanesulfonate, le toluène sulfonate et le trifluoroacétate, et
R⁵ à R⁸ sont identiques ou différents et représentent un reste alkyle ayant 1 à 16 atomes de carbone, un reste aryle ayant 6 à 10 atomes de carbone ou un reste aralkyle ayant 7 à 11 atomes de carbone.

11. Procédé selon l'une quelconque ou plusieurs des revendications précédentes 1 à 10, **caractérisé en ce que** comme sels d'onium, on utilise un sel d'onium quaternaire choisi parmi le chlorure de tétraméthylammonium, le bromure de tétraméthylammonium, le chlorure de tétrabutylammonium, le bromure de tétrabutylammonium, le chlorure de benzyldiméthyltétradécylammonium, le bromure de butyltriphénylphosphonium, le bromure de tétraphénylphosphonium, le bromure de tétrabutylphosphonium et le chlorure de tétrabutylphosphonium.

12. Procédé selon l'une quelconque ou plusieurs des revendications précédentes 1 à 11, **caractérisé en ce qu'**il est conduit en présence d'une base supplémentaire.

13. Procédé selon l'une quelconque ou plusieurs des revendications précédentes 1 à 12, **caractérisé en ce qu'**il est conduit à une température de 50 à 150°C.
